# EUROPEAN PATENT SPECIFICATION

(11) **EP 1 348 127 B1**
(45) Date of publication and mention of the grant of the patent: **17.12.2008**
(21) Application number: 01272578.4
(22) Date of filing: 18.12.2001
(51) Int. Cl.: G01N 33/58, G01N 33/532

(54) **ELEMENTAL ANALYSIS OF TAGGED BIOLOGICALLY ACTIVE MATERIALS**
ELEMENTARANALYTIK VON MARKIERTEN BIOLOGISCH AKTIVEN SUBSTANZEN
ANALYSE ELEMENTAIRE DE MATERIAUX MARQUES BIOLOGIQUEMENT ACTIFS

(30) Priority: 28.12.2000 US 258387 P; 17.07.2001 US 905907
(43) Date of publication of application: 01.10.2003
(73) Proprietor: MDS Inc., doing business as MDS Sciex, Concord, Ontario L4K 4V8 (CA)
(72) Inventor: BARANOV, Vladimir, Richmond Hill, Ontario L4S 1Z4 (CA); TANNER, Scott, Aurora, Ontario L4G 4Z2 (CA); BANDURA, Dmitry, Aurora, Ontario L4G 4A3 (CA); QUINN, Zoe, Toronto, Ontario M5V 2W9 (CA)
(74) Representative: Reinhard - Skuhra - Weise & Partner GbR
(86) International application number: PCT/CA2001/001815
(87) International publication number: WO 2002/054075

(56) References cited:
- US-A- 4 022 876
- US-A- 4 205 952
- US-A- 4 454 233
- US-A- 5 907 034
- BLAKE, D A ET AL: "Immunoassays for metal ions" ANALYTICA CHIMICA ACTA, vol. 376, 1998, pages 13-19, XP001063361
- DARWISH I A ET AL: "ONE-STEP COMPETITIVE IMMUNOASSAY FOR CADMIUM IONS: DEVELOPMENT AND VALIDATION FOR ENVIRONMENTAL WATER SAMPLES" ANALYTICAL CHEMISTRY, AMERICAN CHEMICAL SOCIETY. COLUMBUS, US, vol. 73, no. 8, 15 April 2001 (2001-04-15), pages 1889-1895, XP001030286 ISSN: 0003-2700 cited in the application
- NEILSEN J L ET AL: "Laser ablation inductively coupled plasma-mass spectrometry in combination with gel electrophoresis: A new strategy for speciation of metal binding serum proteins." SPECTROCHIMICA ACTA PART B ATOMIC SPECTROSCOPY, vol. 53, no. 2, February 1998 (1998-02), pages 339-345, XP001068501 ISSN: 0584-8547
- EVANS R DOUGLAS ET AL: "A method for characterization of humic and fulvic acids by gel electrophoresis laser ablation inductively coupled plasma mass spectrometry." JOURNAL OF ANALYTICAL ATOMIC SPECTROMETRY., vol. 15, no. 2, February 2000 (2000-02), pages 157-161, XP001068410 ISSN: 0267-9477
- ZHANG CHAO ET AL: "A novel combination of immunoreaction and ICP-MS as a hyphenated technique for the determination of thyroid-stimulating hormone (TSH) in human serum." JOURNAL OF ANALYTICAL ATOMIC SPECTROMETRY, vol. 16, no. 12, December 2001 (2001-12), pages 1393-1396, XP001068414 December, 2001 ISSN: 0267-9477
- BARANOV, V.I. ET AL: "A sensitive and quantitative element-tagged immunoassay with ICPMS detection" ANALYTICAL CHEMISTRY, vol. 74, no. 7, 1 April 2002 (2002-04-01), pages 1629-1636, XP001068480

## Description

### FIELD OF THE INVENTION

The present invention relates to methods for the detection and measurement of element tagged biologically active material. More particularly, the present invention is directed to detection and measurement of tagged immunoglobulins or antigens using an inductively coupled plasma mass spectrometer.

### BACKGROUND OF THE INVENTION

Various methods are in use for the detection and measurement of biological materials. To date, these determinations are generally facilitated through the use of radiological, fluorescent or enzymatic tags. None of these methods have successfully dealt with elemental tagging of biologically active materials, and in particular immunoglobulins and antigens, followed by detection using atomic mass or optical spectrometry.

The methods used to date have included (1) elemental tagging in immunoassays, (2) elemental tagging using radioisotopes, (3) elemental tagging to enhance fluorescence, (4) immunological detection of elemental species without tagging, and (5) direct elemental tagging for cell uptake studies. We will review each of these areas in turn.

### Elemental tagging in immunoassays

Wang 1984 (US 4,454,233) disclosed the possibility of utilizing a mass spectrometer as a means of immunoassay detection. Wang's method required a cumbersome preliminary set of steps to first prepare a tagged 'mobile unit' which was then conjugated to an antibody/antigen. In the preferred embodiment, the 'mobile unit' was comprised of a latex particle embedded with heavy tagging elements such as Fe, Ni, Cu and Co. Among Wang's reasons for utilizing 'mobile units' were: (1) easy separation of bound reactant from unbound reactant, (2) simultaneous detection of many antigen/antibody complexes owing to the small size of mobile units, and (3) possible utilization of 'unstable' or 'reactive' tags, as tags embedded in the latex would not interfere with the reaction. However, due to the burdensome requirement of preparing mobile units, Wang's method was impractical and has not been used for immunoassay detection.

Immunoassay detection using element tagged immunoglobulins and antigens has also been possible using colloidal gold or extremely small beads of gold (several nanometers in diameter), for example NANOGOLD^{™} particles. Van Banchet and Heppelmann (1995), Wagenknecht et al. (1994), and Wenzel and Baumeister (1995) used colloidal gold to visualize protein structure in the cell and to detect receptor-ligand binding by electron microscopy. However, these assays suffer from lack of quantitation capabilities.

Element tagging has also been used in electrochemical immunoassays followed by polarographic detection of the generated complexes based on the catalytic conversion of a substrate by labeled metal ion or the anodic current of metal labeling Qiu and Song (1996). Similar to the preceding example, the assay lacked quantitation capabilities.

Thus, although elemental tagging has been used in immunoassays, the tagging methods have been cumbersome or were ineffective at quantitation.

### Elemental tagging using radioisotopes

Historically, the most common use of elemental tagging has been the use of radioactive elements. While radioassays remain the method of choice due to their exceptional sensitivity to low levels of analyte, their general use is limited by the restrictions in dealing with radioactive materials.

### Elemental tagging to enhance fluorescence

Recently, elemental tagging has been used to enhance luminescence of fluorescent tags. US 4,637,988 to Hinshaw et al., describes the use of lanthanide metals complexed with fluorescent compounds and chelating agents, that can be used in specific binding assays. U.S. 5,958,783 to Josel et al. describes the use of metal complexes with a charged linker as luminescent groups in fluorescence-based or electrochemiluminescence-based assays.

However, these fluorescent tagging methods suffer from the disadvantages associated with their relatively low sensitivity and resulting problems with quantitative analysis in samples containing low concentrations of target molecules. As well, fluorescence-based methods are limited to the analysis and quantification of only one or at most a few target substances per assay.

### Immunological Detection of elemental species

Blake et al. (1998) and Darwish and Blake (2001) disclosed a method of detection and quantitation of elemental species by complexing elemental species with antibodies that recognize chelated elemental species, using antibodies conjugated with fluorescent tags. However, as outlined above, fluorescence based assays suffer from low sensitivity and are limited to one or a few targets per assay.

### Direct elemental tagging in conjunction with gel electrophoresis

Binet et al. (2001) disclosed a method of determining untagged proteins by separation using gel electrophoresis, followed by laser ablation of the separated spots and detection using mass spectrometry. However, this method has been limited to molecules that are naturally detectable by spectrometry.

Wind et al. (2001), Nagaoka and Maitani (2000) and Baldwin et al. (2001) used chromatography to separate proteins, followed by detection using mass spectrometry. Similarly, Chen et al, 2000 incorporated isotopic tags of 13C, 15N and 2H in proteins before chromatographic separation and detection using organic mass spectrometry. However, separation with chromatography is an added step, which can be onerous.

Thus, if one does not tag, one is limited to what is being assayed and using chromatography for separation adds a step to the process.

### Direct elemental tagging for cell uptake studies

De Llano et al.(1996) and de Llano et al. (2000), disclosed a method of visualizing and measuring the uptake of low density lipoprotein (LDL) tagged with colloidal gold by cells using electron microscopy and mass/atomic spectrometry. However, the biological material was labeled directly and gel electrophoresis was not utilized.

Thus, various methods have been developed for visualizing and analyzing element tagged biologically active compounds. However, they have innate limitations, ranging from handling radioactive waste, to low sensitivity with fluorescence based assays, to detection only capabilities, and to cumbersome preparation or separation steps.

An enormous potential exists for the development of very simple biological assays that take advantage of capabilities offered by elemental tagging coupled with elemental detection using a mass or optical spectrometer. Mass and optical spectrometry offer high sensitivity, accurate quantitation and a wide dynamic range. The use of elements to label biologically active material allows construction of an enormous number of distinguishable tags.

This invention involves bridging the science of biology, and in particular immunology, and analytical atomic mass spectrometry. The invention offers an easy and simple means of tagging biological molecules. Further, it offers excellent detection capabilities, equaling (or surpassing) the sensitivity of radioassays. It offers the safety of florescent based assays, and the added feature of an enormous number of available tags, with the possibility of simultaneous detection of numerous biological complexes. In addition, the handling of the reacted tagged complexes can be crude, as the integrity of the chemical complex need not be preserved in assaying the element.

### SUMMARY OF THE INVENTION

The last two decades have seen the improvement of elemental analysis due to the development of the inductively coupled plasma (ICP) source using mass or optical spectrometry. This has resulted in ultra sensitive spectrometers with high matrix tolerance and means of resoling isotopic and spectral interferences. The present invention has coupled the developments in this field with the continuing need to provide rapid and precise detection and measurement in biological assays.

The present invention provides a method as defined in claim 1.

### BRIEF DESCRIPTION OF THE FIGURES

The invention will now be described in relation to the drawings in which
Figure 1 is a graph illustrating a calibration curve obtained by dilutions of human IgG and immunoreaction with the gold-labeled antihuman antibody with detection and quantitation using ICP-MS.
Figure 2 is graph showing the results of an immunoassay with human IgG and Fab-Au, over a low concentration range with detection and quantitation using ICP-MS.
Figure 3 is a graph showing the results of an immunoassay with human IgG and F'ab-Au, over a high concentration range with detection and quantitation using ICP-MS.
Figure 4 is a bar graph showing the results of measuring endogenous protein in cultured cells with detection and quantitation using ICP-MS.
Figure 5 is a graph showing the detection and quantitation of a specific antigen by ICP-MS.

### DETAILED DESCRIPTION OF THE INVENTION

### DEFINITIONS

As used in this application:
**"analyte"** means any substance being identified and measured in an analysis, and includes but is not limited to elemental species, element species chelate complexes; cells, viruses, subcellular particles; proteins including more specifically antibodies, immunoglobulins, lipoproteins, glycoproteins, peptides, polypeptides; nucleic acids including DNA and RNA; and including peptidic nucleic acids; oligosaccharides, polysaccharides, lipopolysaccharides; cellular metabolites, haptens, hormones, pharmacologically active substances, alkaloids, steroids, vitamins, amino acids and sugars.
**"analyte complex"** means an analyte bound to other molecules or biologically active materials.
**"anima"** means all members of the animal kingdom.
**"atomic mass spectrometer"** means a mass spectrometer that generates atomic ions, and detects atomic ions based on the mass/charge ratio.
**"biologically active material(s)"** means any biological substance found in nature and includes cells, viruses, subcellular particles; proteins including more specifically antibodies, immunoglobulins, lipoproteins, glycoproteins, peptides, polypeptides; nucleic acids including DNA and RNA, and including peptidic nucleic acids; oligosaccharides, polysaccharides, lipopolysaccharides, cellular metabolites, haptens, hormones, pharmacologically active substances, alkaloids, steroids, vitamins, amino acids and sugars.
**"capacitively coupled plasma" (CCP)** means a source of ionization in which a plasma is established by capacitive coupling of radiofrequency energy at atmospheric pressure or at a reduced pressure (typically between 1 and 500 Torr) in a graphite or quartz tube
**"corona"** means a source of ionization in which a conductor (typically a needle) is provided a voltage relative to a counter electrode surface (typically containing an ion sampling aperture) such that the voltage gradient exceeds a critical value to cause ionization of the surrounding gas, but not sufficient to cause sparking.
**"elemental species"** means a molecule containing a metal bound to another atom or group of atoms. For example, selenite (SO₃⁻²), selenate (SO₄⁻²), methylselenocysteine and selenomethionine are elemental species of selenium.
**"element tagged"** means a molecule tagged with a transition element, including a noble metal or lanthanide.
**"elemental tag"** means any transition element, including a noble metal or lanthanide, used to tag the biologically active material or analyte.
**"electrospray"** means a source of ionization in which a liquid sample is nebulized from a tube due to the sufficiently high potential applied, which also provides a charge to the droplet, and in which the resultant charged droplet evaporates and fragments yielding small charged droplets or charged molecular ions.
**"Fab"** means the antigen binding fragment of an antibody obtained by papain reaction with immunoglobulin.
**"Fab"'** means the antigen binding fragment of an antibody. Fab' is obtained by pepsin reaction with immunoglobulin, followed by cleavage of two disulfide bonds.
**"Glow discharge"** (GD) means a source of ionization in which a discharge is established in a low pressure gas (typically between 0.01 and 10 Torr), typically argon, nitrogen or air, by a direct current (or less commonly radiofrequency) potential between electrodes.
**"graphite furnace"** means a spectrometer system that includes a vaporization and atomization source comprised of a heated graphite tube. Spectroscopic detection of elements within the furnace may be performed by optical absorption or emission, or the sample may be transported from the furnace to a plasma source (e.g. inductively coupled plasma) for excitation and determination by optical or mass spectrometry.
**"inductively coupled plasma"(ICP)** means a source of atomization and ionization in which a plasma is established in an inert gas (usually argon) by the inductive coupling of radiofrequency energy. The frequency of excitation force is in the MHz range.
**"lanthanide"** means any element having atomic numbers 58-71. They are also called rare earth elements.
**"MALDI"** means a source of ionization (Matrix Assisted Laser Desorption Ionization) in which ions are produced from a sample mixed with a matrix (typically analyzed in crystalline form) by exposure to laser irradiation, typically at low pressure
**"mass spectrometer"** means an instrument for producing ions in a gas and analyzing them according to their mass/charge ratio.
**"microwave induced plasma" (MIP)** means a source of atomization and ionization in which a plasma is established in an inert gas (typically nitrogen, argon or helium) by the coupling of microwave energy. The frequency of excitation force is in the GHz range.
**"multiplexing"** means using more than one elemental tag for the simultaneous or sequential detection and measurement of biologically active material.
**"noble metal"** means any of several metallic elements, the electrochemical potential of which is much more positive than the potential of the standard hydrogen electrode, therefore, an element that resists oxidation. Examples include palladium, silver, iridium, platinum and gold.
**"optical spectrometer"** means an instrument calibrated to measure either wavelength of light or the refractive index of a prism, and includes atomic emission and atomic absorption spectrometers.
**"plasma source"** means a source of atoms or atomic ions comprising a hot gas (usually argon) in which there are (approximately) equal numbers of electrons and ions, and in which the Debye length is small relative to the dimensions of the source.
**"rare earth metals"** means any element having atomic numbers 58-71. The are also called "lanthanides'.
**"sample"** means any composition of liquid, solid or gas containing or suspected of containing an analyte.
**"transition element"** means any element having the following atomic numbers, 21-29, 39-47, 57-79, and 89. Transition elements include the rare earth elements, lanthanides and noble metals. (Cotton and Wilkinson, 1972, pages 528-530).

There are a number of aspects to the present invention.

Illustrative examples 1 to 10 and 14 to 16 describe labeling of biologically active material which binds to an analyte in a sample. Most often, the biologically active material would be an immunoglobulin or antigen. The element is detected by an atomic mass or optical spectrometer having a source of atoms or atomic ions.

Each of the individual steps is described in Materials and Methods section of this application.

The benefits of this aspect of the invention are that: (1) it allows for the detection of minute quantities of analyte, (2) it allows for multiplexing, saving time, resources and providing for a better analysis of the sample, (3) the analysis is very rapid as there is no need to wait for enzymatic reactions, and measurement time by ICP-OES/MS is shorter than radiological tag measurement, (4) it has a large dynamic range, (5) radioisotopes are not required, producing a safe work environment and avoids toxic waste, and (6) the reacted complex does not need to be preserved, allowing the use of acidic media to degrade the complex and stabilize the element in solution and thereby increasing the period of storage of the sample before analysis.

Illustrative examples 11 to 13 describe the determination of elemental species. In cases where mass spectrometry can not differentiate elemental species, the use of antibodies to detect elemental species coupled with mass spectrometry allows for their differentiation.

In this aspect, as show in Example 13, multiplexing can be used. Again, the benefits of this aspect are that: (1) it allows for the detection of minute quantities of analyte, (2) it allows for multiplexing, (3) the analysis is very rapid, (4) there is a large dynamic range, (5) one can avoid the use of radioisotopes, (6) the reacted complexes do not need to be preserved, and (7) chromatographic separation is not required, which speeds up and simplifies the analysis.

Example 17 describes the direct labeling of the analyte. The individual steps involved in this third aspect are known to those skilled in the art, but direct labeling coupled with mass spectrometry is new and inventive. A variation of this aspect is described in Example 9. Again, the benefits of this third aspect are that: (1) it allows for the detection of minute quantities of analyte, (2) it allows for multiplexing, (3) the analysis is very rapid, (4) there is a large dynamic range, (5) one can avoid the use of radioisotopes, (6) the reacted complexes do not need to be preserved, and (7) chromatographic separation is not required, which speeds up and simplifies the analysis.

For all aspects of the invention, it is understood that the biologically active material can be added to the analyte, or the analyte can be added to the biologically active material. Further, an analyte complex can be formed, by the binding of molecules to the analyte, as seen in the examples outlined below, in which a series of antibodies (primary, secondary, tertiary) can be conjugated to the analyte.

### Tagging Elements

The choice of the element to be employed in the methods of the present invention is preferably selected on the basis of its natural abundance in the sample matrix under investigation. In order to achieve selectivity, specificity, the ability to provide reproducible results, and include appropriate standards for accurate quantitation, it is evident that the tagging element should be of low natural abundance. For example, in a preferred embodiment, the rare earth elements or gold can be used as tag materials. Yet, in another embodiment, an unusual isotope composition of the tag can be used in order to distinguish between naturally present elements in the sample and the tag material. In this case non-radioactive isotopes of, for example, iron, potassium, nickel or sodium can be successfully distinguished from naturally abundant isotopes employing the elemental analysis.

The size of an elemental tag (ratio of atoms which are detectable by means of the elemental analysis to the each antigen-antibody conjugate) may be varied in order to produce the most consistent, sensitive and quantitative results for each particular set of antigens and antibodies.

In a preferred embodiment of this invention, several conjugates can be used in one sample simultaneously providing that the tagging material was selected to be different in every assay. In this embodiment the preferred ICP-MS technique is used in order to quantify different tagging elements simultaneously or sequentially depending on the apparatus employed.

Although many applications of the present method will involve the use of a single elemental tag for each antibody or antigen, it should be readily appreciated by those skilled in the art that an antibody or antigen may be tagged with more than one element. As there are more than 80 naturally occurring elements having more than 250 stable isotopes, there are numerous elements, isotopes and combinations thereof to choose from.. For example, there are 20 distinguishable 3-atom tags that may be constructed from only 4 different isotopes, and one million distinguishable 15-atom tags that may be constructed from 10 different isotopes, or 70-atom tags that may be constructed from 5 different isotopes. Within limits prescribed by the need to have distinguishable tags when in combination, this will allow for simultaneous detection of numerous biologically tagged complexes. It is advantageous if the relative abundance of the tag elements is sufficiently different from the relative abundance of elements in a given sample under analysis. By "sufficiently different" it is meant that under the methods of the present invention it is possible to detect the target antibody or antigen over the background elements contained in a sample under analysis. Indeed, it is the difference in inter-elemental ratios of the tagged antibody or antigen, and the sample matrix that can be used advantageously to analyze the sample.

It is feasible to select elemental tags, which do not produce interfering signals during analysis. Therefore, two or more analytical determinations can be performed simultaneously in one sample. Moreover, because the elemental tag can be made containing many atoms, measured signal can be greatly amplified.

### Detection of Metal Ions and elemental species

As was indicated above, an important application of the method of the present invention is the detection of metal in samples, such as toxic metals in environmental settings, including organisms, animals, and humans. Preferably, the invention detects metals in environmental settings. However, as is readily apparent to those skilled in the art, the toxicity of metals depends on the oxidation state, and often on the chemical structure of the elemental species. While an elemental detector, such as uses an ICP source, is able to determine the total quantity of an element in a sample it is generally unable to distinguish different species. There is an ongoing attempt to use different forms of chromatography to pre-separate the sample before the ICP, but this approach has been plagued with concern about the integrity of the sample, i.e., preservation of the oxidation state during sample preparation. The method of the present invention provides a means by which a long-standing problem of detecting speciation is overcome.

In a further embodiment of the present invention, there is provided a method of determining the concentration of a metal ion of interest, preferably toxic metals, more preferably in environmental/biological samples, comprising preparing an antibody which is specific to a selected speciation state of a given toxic metal, reacting said antibody with a solution suspected of containing a toxic metal, and detection of the resulting complexes by application of ICP- MS. Methods for the preparation of an antibody which is specific to a selected oxidation state of a given toxic metal are known by those skilled in the art and are described, for examples, in Bosslet et al.(1999), Blake et al. (1998), and Bordes et al. (1999).

In a further embodiment of the present invention, a tagged antibody is added to a sample containing a speciated element. The sample is split into two halves. The first half of the sample is analyzed for total speciated element. In the second half of the sample, the reacted complexes are separated from the unreacted. The tagging element and the speciated element are quantified in the reacted sample. The speciated element is also quantified in the unreacted sample. In this instance, the results will provide complementary data, and the fraction of the specific species in question will be determined.

As was also indicated above, an important application of the method of the present invention is the detection of elements of tags in samples by means of laser ablation of polyacrylamide gels where tagged molecules are separated by electrophoresis. This application can be used in order to analyze biomolecules in gels rapidly without destroying the sample. Also, by employing microablation it is feasible to distinguish cancerous cells from normal cells on histological section of biopsy samples using element-tagged antibodies specifically attached to the markers of cancerous populations.

The following section describes the methods and materials required to carry out the following invention.

### METHODS AND MATERIALS

### ICP-MS Techniques

Techniques using ICP-MS or OES can be applied for the purposes of this invention.

For example, in its latest realization it was described in Tanner el al.(2000a), Baranov et al. (1999), Tanner et al. (1999), Tanner et al. (2000b), and Bandura *et al.* (2000). This successful modification of ICP-MS includes the dynamic reaction cell, which is used in order to reduce isobaric interferences in atomic mass spectrometry. Briefly, the ICP-DRC-MS technique comprises a high temperature plasma in which the sample particles are atomized and ionized; vacuum interface which is designed to transport the plasma together with analyte ions from atmospheric pressure to vacuum; ion focusing optics; the dynamic reaction cell for chemical modification of the ion current and mass analyzing devise (quadrupole, TOF or magnetic sector). The sample is usually introduced to the plasma as a spray of droplets (liquid sample) or flow of particles (laser ablation of solid surfaces).

### Sources of atoms and atomic ions

The source of atoms or atomic ions can be produced from the following sources: inductively coupled plasma (ICP), graphite furnace, microwave induced plasma (MIP), glow discharge (GD), capacitively coupled plasma (CCP), electrospray, MALDI or corona.

### Antibody Preparation

According to a preferred embodiment of the methods of the present invention, elementally tagged antibodies, or antibodies directed to a metal of interest are employed. Antibodies that bind a target of interest can be prepared using techniques known in the art such as those described by Kohler and Milstein (1975), Wakabayashi et al. (1990), Frackelton et al. (1985) and Gillis (1983), which are incorporated herein by reference. (See also Kennett, McKearn, and Bechtol (1980), and Harlow and Lane (1988), which are also incorporated herein by reference).

Within the context of the present invention, antibodies are understood to include monoclonal antibodies, polyclonal antibodies, antibody fragments (e.g., Fab, and F(ab')₂) and recombinantly produced binding partners. Antibodies are understood to be reactive against the target antigen if they bind to the target with an affinity of greater than or equal to 10⁻⁶ M.

### Tagging of Biologically Active Materials

Preferably, the tagging element is in the form of a nanoparticle, which is attached to a biologically active material, such as for example an antibody, without degrading its activity (tagged conjugate). Examples of techniques for coupling elemental tags to biologically active materials are well known to those skilled in the art. For example, Barlett, P. A. et al. (1978), describe a metal cluster compound (Au₁₁) having a core of 11 gold atoms with a diameter of 0.8 nm. The metal core of 11 gold atoms in the undecagold metal cluster compound is surrounded by an organic shell of PAr₃ groups. This metal cluster compound has been used to form gold immunoprobes, for example, by conjugating Au₁₁ to Fab' antibody fragments as well as other biological compounds.

Another metal cluster compound which has been used as a probe is Nanogold^{™}. Nanogold^{™} has a metal core with 50-70 gold atoms (the exact number not yet being known but believed to be 67 gold atoms) surrounded by a similar shell of organic groups (PAr₃) as undecagold. The metal core of Nanogold^{™} is 1.4 nm in diameter.

A more recent description of techniques for the preparation of biological tags which may be used in the method of the present invention is found in Hainfeld *et al.* (1996) (US 5,521,289). Briefly Hainfeld *et al.* (1996) describes, among others, thiol gold clusters produced by forming an organic-gold complex by reacting a compound containing a thiol with gold in solution. A second equivalent is also added of the thiol compound. Finally the gold organic is reduced with NaBH₄ or other reducing agents and organometallic particles are formed. These have the general formula Auₙ R.ₘ R'₁ ..., where n, m, and 1 are integers, R and R' are organic thiols, (e.g., alkyl thiols, aryl thiols, proteins containing thiol, peptides or nucleic acids with thiol, glutathione, cysteine, thioglucose, thiolbenzoic acid, etc.). With two equivalents of organic thiol compound, clusters with gold cores ∼1.4 nm are formed with many organics. The organic moiety may then be reacted by known reactions to covalently link this particle to antibodies, lipids, carbohydrates, nucleic acids, or other molecules to form probes. Mixtures of organic thiols may be used to provide mixed functionality to the clusters. These organo-gold clusters are stable to heating at 100 degrees C.

These organic thiol-gold preparations may also be made using similar processes with alternative metals to gold, e.g., platinum, silver, palladium and other metals, or mixtures of metal ions, e.g., gold and silver, resulting in mixed metal clusters. The metal clusters together with all other components of a sample are readily atomized and ionized in the high temperature ICP for subsequent MS or OES analysis.

### Separation Techniques

According to one embodiment of the present invention, a tagged conjugate may be isolated for analysis by employing a filtration technique. For Example, after incubation of an antigen with the tagged conjugate the sample undergoes filtering through a size separating centrifugal filter. Non-reacted tagged antibody together with other components of the sample mixture including non-reacted antigen pass into the filtrate. Complexes of antigen and antibody conjugate are left on the filter and after washing out can be stabilized in acidic solution. Since the integrity of the sample (i.e. the chemical form) is not important after separation, the separated sample can be acidified/degraded/stabilized (for example in acidic media) and quantitative analysis is preferably carried out using the ICP-MS technique. The optimal concentrations of all reagents for each system should be determined in an initial criss-cross serial dilution experiment and the concentration of reagent being quantitated must lie within the dynamic range of the standard curve. As will be readily apparent to those skilled in the art, other techniques of separation of free substance or non-complexed proteins from complexed substance may be used, for examples, salting out, chromatography, electrophoresis, gel filtration, fractionation, absorption, polyacrylamide gel electrophoresis, agglutination, or combinations thereof.

In light of the present disclosure, those skilled in the art will readily appreciate other methods and applications of the methods of the present invention.

### EXAMPLES

### Example 1. Nanogold^{™} immunoassay

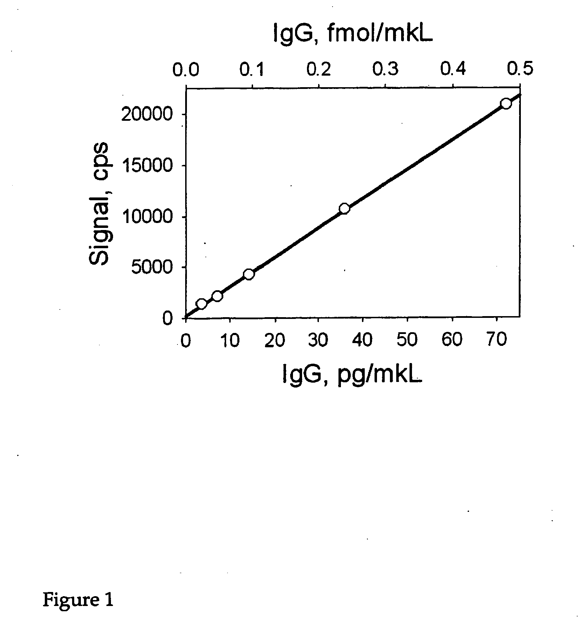

The following provides an example of the methods of the invention using the Nanogold-IgG^{™} (or Nanogold-Fab'^{™}; Nanoprobes) immunoassay and its protocol. PBS buffer A is prepared as follows: 150mM NaCl; 20mM phosphate, pH 7.4; 1% BSA (bovine serum albumin). All eppendorf tubes, micro-titer plates, and filters to be used subsequently are pacified with PBS buffer A for 1 hour at room temperature. This treatment will reduce the non-specific interactions that occur between the plastic used and the antigen and Nanogoid-IgG^{™}. Alternatively low retention plastic products can be used (e.g. Axygen tubes). Following this a solution of the antigen (peptide, protein, etc.) in the concentration range of 1000 to 0.5 pg/µl in PBS buffer A is prepared. All dilutions are stored on ice. Subsequently, 100ul of either antigen solution or PBS buffer A (for controls) is pipetted into the individual wells of the micro-titer plate (or set of eppendorf tubes). The Nanogold-IgG^{™} is pre-filtered through 300KDa (MICRCON^{™} or Centricon^{™}) centrifugal filter devices. Dilutions of filtered Nanogold-IgG^{™} in PBS buffer A are prepared as follows: A 1:50 dilution is produced by adding 60µl of Nanogold-IgG^{™} in 2940µl PBS buffer A. A 1:500 dilution is then produced by adding 100µl of Nanogold-IgG^{™} to 900µl of PBS buffer A. Depending on concentration range of antigen, 100 to 500 µl of 1:500 Nanogold-IgG^{™} is then added to the wells of the plate and then incubated for 1-2 hours at room temperature. The total amount of antigen- Nanogold-IgG^{™} mix is then pipetted into the sample reservoir (upper chamber) of a 300KDa MICROCON^{™} centrifugal filter device (max volume 2ml). This sample is centrifuged at 14,000g for 15 minutes at room temperature. The assembly is removed from the centrifuge and the vial separated from sample reservoir. The sample reservoir is inverted in a new vial, and spun for 3 minutes at 1000g to transfer the concentrate to a new vial. Finally, a fixed volume of the collected antigen-Nanogold-IgG^{™} antibody mixture is diluted to 1ml with 10%HCl/1ppbIr for stabilization. Ir provides an internal standard for ICP-MS quantitation and the acid solution is suitable for the elemental analysis. The linearity of the ICP-MS detector response as a function of the concentration of the analyte human IgG is shown in the results presented in Figure 1.

### Example 2. Immunoassay, other than Nanogold-IgG^{™}.

According to this example, an antibody is tagged with an element (eg. Eu, Ru, etc.) suitable for analysis by ICP-MS and is introduced into a sample containing antigens of interest (e.g. human blood proteins). The element-tagged antibody reacts specifically to the target antigen. The resulting tagged antigen-antibody complex is separated from un-reacted antibody (as in Example 1, 3, 4, 5, 8, 9, or 10), and the tagged complex is analyzed by ICP-MS. Variations of this example include:
a) Tagging with multiple atoms to amplify the signal and thereby improving detectability.
b) As, a), except the tag contains several isotopes of the same element or different elements, preferably in a non-natural (unusual) distribution, so that the unique isotope distribution is a determinant of the targeted antigen. It is to be recognized that there are more than 80 naturally occurring elements) of which some 60 may have value in this application) having more than 250 stable isotopes. This allows construction of an enormous number of distinguishable tags. For example, there are 20 distinguishable 3-atom tags that may be constructed from only 4 different isotopes, and one million distinguishable 15-atom tags from 10 different isotopes, or 70-atom tags from 5 different isotopes.
c) As in a) and b), but incorporating different antibodies with specificity to different target molecules, to allow simultaneous determination of different target molecules. The number of simultaneous determinations is limited by the number of distinguishable tags in combination (which is fewer than the number of distinguishable tags in isolation as described above).

### Example 3. Protein A Sepharose Immunoassay

The following provides an example of the methods of the invention using the Protein A Sepharose CL-4B^{™} (Pharmacia) immunoassay and its protocol. Either Nanogold-Fab'^{™} or another element-labeled Fab' specific to the target protein (or host species of the secondary antibody) may be used. There are two types of immunoassays that may be used:
a) Direct immunoassay, which would involve trapping the target protein of interest (protein X) by incubating Protein A Sepharose CL-4B^{™} with an excess of antibody specific to the target protein, washing off the un-reacted antibody, adding the protein sample, washing off the unbound protein, and then exposing the PAS-antibody-protein X complexes to element-labeled, anti-X Fab'.
b) Indirect immunoassay, which would involve trapping the target protein of interest (protein X) by incubating Protein A Sepharose CL-4B^{™} with an excess of primary antibody (e.g. polyclonal) specific to the target protein, washing off the un-reacted primary antibody, adding the protein sample, washing off the unbound protein, and then exposing the PAS-antibody-protein X complexes to a second antibody specific to protein X (e.g. a monoclonal antibody), washing off un-reacted secondary antibody, and then incubating the PAS-antibody-protein X-antibody complexes with an element-labeled, anti-secondary Fab'. Alternatively beads or micro-titer plates covalently bound to anti-protein X antibodies may be used.

PBS buffer A is prepared as follows: 150mM NaCl; 20mM phosphate, pH 7.4; 1% BSA (bovine serum albumin). All eppendorf tubes, micro-titer plates, and filters, and slurry of Protein A Sepharose CL-4B^{™} to be used subsequently are pacified with PBS buffer A for 1 hour at room temperature. This treatment will reduce the non-specific interactions that occur between the plastic used and the antigen and Nanogold-Fab'^{™}. Alternatively low retention plastic products can be used (e.g. Axygen tubes). Following this a solution of the antigen (peptide, protein, etc.) in the concentration range of 1000 to 0.5 pg/µl in PBS buffer A is prepared. All dilutions are stored on ice. Subsequently, 100ul of either antigen or PBS buffer A (for controls) is pipetted into the individual wells of the micro-titer plate (or set of eppendorf tubes). The Nanogold-Fab'^{™} is pre-filtered through 300KDa MICRCON^{™} (or Centricon^{™}) centifugal filter devices. Dilutions of filtered Nanogold-Fab'^{™} in PBS buffer A are prepared as follows: A 1:50 dilution is produced by adding 60µl of Nanogold-Fab'^{™} in 2940µl PBS buffer A. A 1:500 dilution is then produced by adding 100µl of Nanogold-Fab'^{™} to 900µl of PBS buffer A. Depending on concentration range of antigen, 100 to 500 µl of 1:500 Nanogold-Fab'^{™} is then added to the wells of the plate and then incubated for 1-2 hours at room temperature. The sample is centrifuged at 14,000rpm for 2 minutes at room temperature. The beads are washed four times with PBS buffer A. In method b) the additional steps to include consist of washing off un-reacted monoclonal antibody, and then incubating the PAS-antibody-protein X-antibody complexes with an element-labeled, anti-X Fab'. Finally, a fixed volume of 10%HCl/1ppbIr is added to each well. Ir provides an internal standard for ICP-MS quantitation and the acid solution is suitable for the elemental analysis.

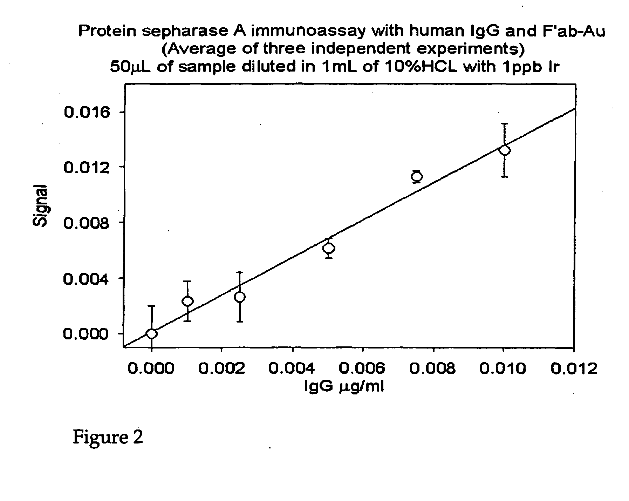

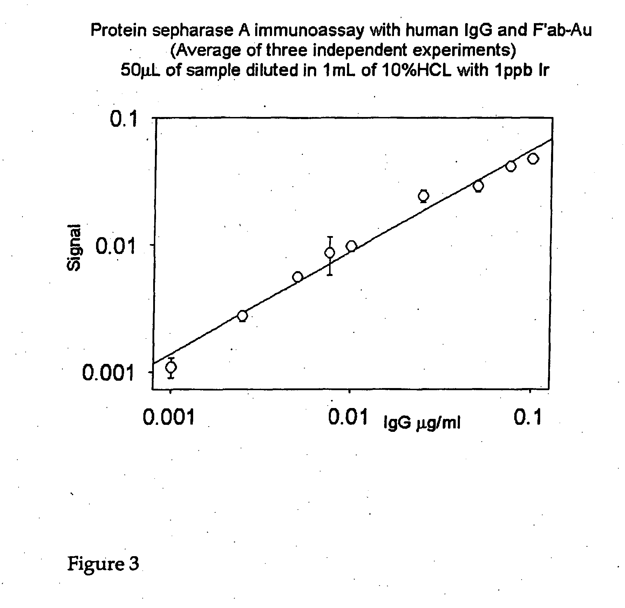

Experimental results obtained according to method a) are given in Figures 2 and 3, using human IgG as the analyte with F'ab-Au as the tagged antibody. Figure 2 provides the calibration results over a relatively low concentration range, and Figure 3 over a higher concentration range. Together, the data exhibit greater than 2 orders of magnitude of detector linearity with respect to the analyte concentration.

This Example also permits multiplexing to be analyzed and can be used to identify protein-protein interactions. In this method, cell lysate is collected and subjected to the method as above where an interaction is suspected between protein A and protein B. In this case the primary antibody would be specific to protein A and an element-labeled Fab' would be specific to protein B. Interactions with multiple other proteins (e.g. protein C and protein D) could be detected at the same time, providing that different elements were used to label anti-Fab' specific to protein C and anti-Fab' specific to protein D.

### Example 4. Dynabeads^{™} Immunoassay

The following method provides an example of the invention using the Dynabeads^{™} (Dynal) immunoassay and its protocol. This immunoassay is performed as in Example 3, using Dynabeads^{™} in place of Protein A Sepharose CL-4B^{™}. Instead of centrifuging the sample, the sample is exposed to a magnetic device (Dynal UTC^{™}). This draws the beads to the bottom of the wells between and after each wash step. Again, 10%HCl/1ppbIr is added to each well in the final step to provide an internal standard for ICP-MS quantitation and elemental analysis.

In the same manner as described for Example 3, multiplexing and protein-protein interactions can be identified using this method.

### Example 5. Method for detection and quantification of endogenous proteins in cultured cells.

There are two methods by which the discrete changes in the levels of endogenous proteins in culture cells can be measured.
a) Direct immunoassay, in which an antibody specific to the protein of interest is required. This antibody is labeled with an element suitable for analysis by ICP-MS.
b) Indirect immunoassay, in which an antibody (primary antibody) specific to the protein of interest is required. In addition a secondary antibody specific to the primary antibody is labeled with an element suitable for analysis by ICP-MS.

A mono-layer of attached cultured cells is grown and treated with conditions of interest. The growth media is removed and the cells are washed with 1xPBS three times. PBS is then replaced with ice-cold methanol and the culture dishes are incubated at -20°C for 5 minutes. The methanol is removed and the cells are allowed to dry completely. A blocking buffer (e.g. 10% horse serum, 1% BSA, 0.05% Tween-20, 1xPBS) is added to the culture dishes and the dishes are incubated for 1-2 hours at room temperature. In method a) an antibody specific to the protein of interest is labeled with an element, diluted in blocking buffer and added to the culture dishes. The cells are exposed to the antibody mix for 2 hours at room temperature (or 37°C). During this time, the element-tagged antibody binds the target protein through its tail. In method b) the antibody specific to the protein of interest is not labeled. In the next step, the un-reacted primary antibody is washed away with blocking buffer. In method b) the element-labeled secondary antibody is diluted in blocking buffer and applied to the cells. The dishes are incubated for 1-2 hours at room temperature. The un-reacted secondary antibody is then washed away with blocking buffer. Finally, in both methods, an acid solution (e.g. concentrated HCl) is added, to release and dissolve the tagging element. The dissolved element in acid is diluted with 10% HCl/1ppb Ir to provide an internal standard. The acid solution containing the tagging element is then analyzed by ICP-MS to quantify the protein of interest.

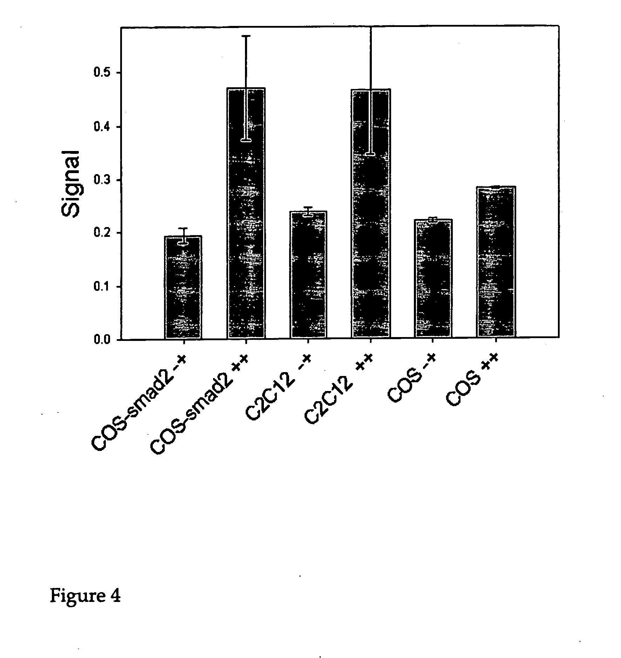

Experimental data obtained according to method b) is shown in Figure 4. This data examines the sensitivity of this immunoassay, by comparing the relative amounts of Smad2 in three different cell cultures; COS (Bars 5 and 6), COS transfected with pCMV5B-Smad2 (COS-smad2) (Bars 1 and 2), and C2C12 cells (Bars 3 and 4). COS cells are known to have undetectable levels of Smad2 protein (using Western blot analysis). Conversely, Smad2 is detectable in C2C12 cell lysate and in COS cells that have been transfected with pCMV5B-Smad2. These cell cultures are prepared in 60mm dishes, fixed with methanol, blocked with TBST buffer and then incubated in either the presence (Bars 2, 4, and 6) or absence (Bars 1, 3, and 5) of polyclonal anti-Smad2 antibody (Upstate Biotech). Cells are incubated with a gold-tagged anti-rabbit antibody (Nanoprobes), dissolved in concentrated HCl, diluted 2 fold in 10%HCl/1ppb Ir and analyzed using the ICP-MS. Each bar is an average of triplicate samples. Bars 1, 3, and 5 reflect negative control cultures not treated with primary antibody (-+). Cultures treated with both primary and secondary antibodies (++) show that in the two cell cultures that express smad2, a substantial increment in the signal over the (-+) results indicates the presence of the smad2 protein. The third culture, COS, which is not expected to express smad2, shows a signal for the (++) case that is roughly comparable to that of the blank (-+).

### Example 6. Method for determination efficiency of cell transfection.

The effectiveness of cell culture transfection is determined by first modifying cells to transduce a tail (e.g. FLAG^{™}). As in Example 5, there are two methods by which the antigen of interest can be detected (directly and indirectly).
a) Direct immunoassay, in which an antibody specific to the tail is required. This antibody is labeled with an element suitable for analysis by ICP-MS.
b) Indirect immunoassay, in which an antibody (primary antibody) specific to the tail is required. In addition a secondary antibody specific to the primary antibody is labeled with an element suitable for analysis by ICP-MS.

Between 1-3 days after transfection of the cells, the growth media (typically 10%FBS, depending on cell-type) is removed and the mono-layer of attached cells are washed with 1xPBS three times. PBS is replaced with ice-cold methanol and the culture dishes are incubated at -20°C for 5 minutes. The methanol is removed and the cells are allowed to dry out completely. A blocking buffer (e.g. 10% horse serum, 1% BSA, 0.05% Tween-20, 1xPBS) is added to the culture dishes and the dishes are incubated for 1-2 hours at room temperature. In method a) an antibody specific to the tail is produced and labeled with an element that is suitable for analyzing with the ICP-MS. The antibody is diluted in blocking buffer and added to the culture dishes. The cells are exposed to the antibody mix for 2 hours at room temperature (or 37°C). During this time, the element-tagged antibody binds the target protein through its tail. In method b) the antibody specific to the protein of interest is not labeled. The unreacted primary antibody is washed away with blocking buffer. In method b) the element-labeled secondary antibody is diluted in blocking buffer and applied to the cells. The dishes are incubated for 1-2 hours at room temperature. The un-reacted secondary antibody is washed away with blocking buffer. Finally, in both methods, an acid solution (e.g. concentrated HCl) is added, to release and dissolve the tagging element. The dissolved element in acid is diluted with 10% HCl/1ppb Ir to provide an internal standard. The acid solution containing the tagging element is analyzed by ICP-MS to quantify the efficiency of the transfection. Culture dishes containing non-transfected cells cultured at the same time can be used as a negative control An alternate variation of this Example involves using a 6xHIS-tagged construct^{™} (Invitrogen), where there is no need for specific antibodies. Cells transfected with 6xHIS-tagged constructs are fixed with methanol, blocked with the blocking buffer and incubated for 2 hours with a solution containing nickel (e.g. Ni-NTA^{™}; Qiagen). The cells are washed to remove free nickel, acid degraded, and analyzed using ICP-MS for nickel content.

### Example 7. Reporter assay

In the study of transcription factors, it is necessary to quantitate the levels of transcription. There are two methods by which discrete changes in the levels of transcription activity on a specific promoter (or enhancer elements) can be measured. Cultured cells are transfected with expression plasmids of interest along with equal amounts of plasmid containing the promoter of interest linked to a reporter gene (e.g. GFP). As in Example 5 there are two methods by which the antigen of interest can be detected (directly and indirectly).
a) Direct immunoassay, in which an antibody specific to the reporter is required. This antibody is labeled with an element suitable for analysis by ICP-MS.
b) Indirect immunoassay, in which an antibody (primary antibody) specific to the reporter is required. In addition a secondary antibody specific to the primary antibody is labeled with an element suitable for analysis by ICP-MS.

Cultured cells are grown and transfected with conditions of interest. Upon analysis; the growth media is removed and the cells are washed with 1xPBS three times. PBS is replaced with ice-cold methanol and the culture dishes are incubated at -20°C for 5 minutes. The methanol is removed and the cells are allowed to dry out completely. A blocking buffer (e.g. 10% horse serum, 1% BSA, 0.05%. Tween-20, 1xPBS) is added to the culture dishes and the dishes are incubated for 1-2 hours at room temperature. In method a) an antibody specific to the reporter is labeled with an element, diluted in blocking buffer and added to the culture dishes. The cells are exposed to the antibody mix for 2 hours at room temperature (or 37°C). During this time, the element-tagged antibody will bind the reporter. In method b) the antibody specific to the reporter is not labeled. In the next step, the un-reacted antibody is then washed away with blocking buffer. In method b) the element-labeled secondary antibody is diluted in blocking buffer and applied to the cells. The dishes are incubated for 1-2 hours at room temperature. The un-reacted antibody is then washed away with blocking buffer. Finally, in both methods, an acid solution (e.g. concentrated HCl) is added, to release and dissolve the tagging element. The dissolved element in acid is diluted with 10% HCl/1ppb Ir to provide an internal standard. The acid solution containing the tagging element is analyzed by ICP-MS to quantify the protein of interest.

### Example 8. Detection of proteins after electrophoresis using tagged antibodies.

A sample of proteins is diluted in 2xSDS sample buffer (1% SDS, 2% glycerol, 100mM Tris, pH6.8, 5% β-mercaptoethanol, 1% DTT, 1% PMSF, 0.2% leupeptin, 0.2% pepstatin) and exposed to electrophoresis on a 2-D or polyacrylamide gel (SDS-PAGE or N-PAGE) to separate the proteins. The proteins from the gel are transferred to nitrocellulose using a semi-dry transfer apparatus (or equivalent). The nitrocellulose is blocked for 1 hour at room temperature using a blocking buffer (e.g. 5% milk in 1xPBS). An element-tagged antibody that recognizes the target protein is added to blocking buffer and the nitrocellulose blot is exposed to the antibody-containing buffer for 2 hours at room temperature. Alternatively an un-labeled primary antibody that recognizes the target protein is used to bind the target protein, followed by washes with wash buffer, and then probing with a secondary anti-primary antibody that is labeled with an element. The nitrocellulose blot is washed three times with wash buffer (0.2%NP40 in 1xPBS). The protein in question is analyzed and quantified by laser ablation.

### Example 9. Detections of proteins after modification with 6xHIS-tag^{™} (Invitrogen) and separation by electrophoresis.

This Example is similar to Example 8; however, the proteins in the sample are modified prior to electrophoresis so that they have an affinity for an element (e.g. the 6xHIS modification yields affinity to Nickel). The gel or blotting paper containing the separated proteins is washed with a solution containing an element (e.g. Ni) that is bound by the protein modification: The gel or blotting paper is analyzed by laser ablation (or direct excisions) and ICP-MS.

### Example 10. Size Exclusion Gel Filtration Immunoassay

In this example, ICP-MS is used to detect the presence of a specific antigen. Accordingly, an antibody is tagged with an element (eg. Au, Eu, Ru, etc.) and is introduced into a sample containing the antigen of interest. The elemental-tagged antibody reacts specifically to the target antigen. The resulting tagged antigen-antibody complex is separated from un-reacted antibody using gel filtration (e.g. HiPrep Sephacryl^{™}; Pharmacia) in a running buffer containing 1ppbIr. The eluate is collected in 0.5ml increments into a 96 well plate, diluted in acid, and analyzed by ICP-MS.

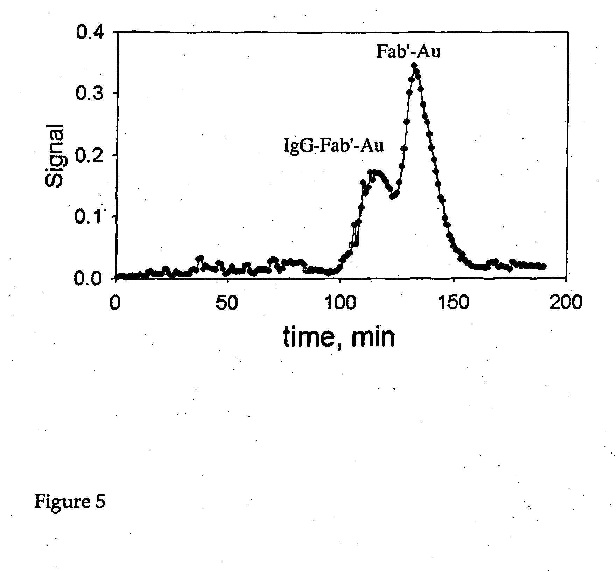

Experimental results obtained according to this method for IgG analyte using Fab'-Au antibody are shown in Figure 5. In this experiment an IgG analyte is incubated with an excess of Fab'-Au. The sample is run through a sephacryl S-200 column at a flow rate of 0.5ml/min, using a running buffer of 0.15M NaCl, 0.02M phosphate, pH 7.4, 1ppb Ir. The figure provides the detector response as a function of elution time (eluate number). The first peak observed (the heavier molecular weight) corresponds to the reacted complex, having an expected molecular weight of about 235 kDa. The second peak corresponds to the unreacted tagged antibody having an expected molecular weight of about 85 kDa.

### Example 11. Detection and quantitation of elemental species.

In this example, ICP mass spectrometry is used to measure a quantity of metal identified by an antibody which is specific for a given oxidation state, molecular form or species of a given metal. A target sample containing cationic metal ions is mixed with a molar excess of a metal-free chelator. A high affinity chelator is chosen to ensure that all cationic metal ions in the sample form a complex with the chelator. This solution is then incubated with an antibody, which is specific for the chelator-metal complex. This solution is treated to separate antibody-metal-chelator complexes from un-reacted antibody and the remainder of components in the sample, although it is important only that extraneous metals be removed from the sample.

Preferably, the antibody exhibits little or no ability to bind to the metal-free chelator, and exhibits a tight and specific binding of the metal-complex which is to be measured. Preferably, this binding affinity shows an equilibrium dissociation constant (K_{D}) on the order of 10⁻⁹ to 10⁻⁸M. The antibody used in such assays also is able to resist interference from other components contained in the sample, which is being assayed. The generation of such antibodies may be carried out according to the procedure described in Blake et al. (1998).

The solution containing the antibody-metal-chelator complexes is subject to standard ICP-MS/OES analysis. This approach removes the necessity for a chromatographic pre-separation and consequently improves the sample integrity. It also allows for simultaneous measurement of several metals/oxidation states/elemental species, the method being limited only by the number of antibodies introduced to the sample.

### Example 12. Detection and quantitation of elemental species using tagged antibodies.

According to this Example, as in Example 11, antibodies specific for metal-chelator complexes are raised according to methods well known to those skilled in the art. The difference in this Example is the antibody is tagged with multiple atoms of a given tagging isotope, or a stoichiometric mixture of isotopic tags. This has two potential advantages. First, in the event that the target metal element is subject to interference in analysis through typical ICP-based interferences (for example argide ion isobaric interferences) tagging the antibody with a normally non-interfered tag allows for interference-free determination, resulting in improved detectablity. Secondly, specific tags for various species of the same target element allows simultaneous measurement of various species (which would not be provided if the elemental tag were the innate target element itself, since the presence of that element in the spectrum would indicate only that one or more of the target species is present). A further advantage according to this approach is that tagging with multiple atoms of the same isotope allows for signal amplification proportional to the number of atoms of the same tagging isotope.

### Example 13. Simultaneous detection of numerous elemental species in a sample using tagged or untagged antibodies.

According to this example, as in Example 11 and 12, antibodies specific for metal-chelator complexes are raised according to methods well known to those skilled in the art. The difference in this Example is that two or more antibodies specific to different elemental species are incorporated, to allow for the simultaneous determination of different speciation states of the same or different elements (where each element is differentially tagged).

### Example 14. Immunoassay to detect Bovine Spongiform Encephalopathy (BSE) in animal products.

The methods of Examples 1, 2, 3, 4, 5, 8, and/or 10 are employed to detect BSE in animal products. There are several monoclonal antibodies (15B3, Korth *et al.,* 1997; KG9, Laffling *et al.,* 2001; Bio-Rad Laboratories) that have been produced that target the prion protein PrP thought to be the infectious component responsible for the illness. Monoclonal antibodies specific to PrP are labeled with an element (eg. Au, Eu, Ru, etc.) and used in immunoassays described in either Example 1, 2, 3, 4, 5, 8, and/or 10. Similar products known to be free of BSE would be used as a negative control. In a similar manner other diseases detected for by antibody can be screened for (e.g. HIV, HTLV, Rabies, etc.).

### Example 15. Immunoassay to detect ischemic markers in patients believed to have suffered a heart attack

The methods of Examples 1, 2, 3, 4, 5, 8, and/or 10 are employed to simultaneously detect multiple ischemic markers in human samples. Candidate markers include: CK-MB, myoglobin, Troponin I, hsp70, BCL2, Bax, IGF, TNFα, angiostatin II.

### Example 16. Method for Drug Discovery

In order to aid in drug discovery, animal cells or animal receptors are placed in multi-well plates. The molecule of interest is added (i.e. potential drug), as well as element-tagged antibody (or element tagged ligand) that recognizes the receptor. The potential drug is in competition with the antibody for adhesion to the receptor. Unbound antibody is washed away, and the amount of bound antibody is determined by ICP-MS. This is inversely proportional to the effectiveness of the potential drug to recognize the receptor. If each well is provided with differently labeled antibodies, then by combining the contents of the wells, one can simultaneously assess the effectiveness of various drugs, or drug compositions by deconvoluting the resultant data. Likewise, differently labeled antibodies for the same analyte can be produced and placed in corresponding wells of different plate (i.e. 10 differently labeled version of the antibody, each one placed in well 1, 1 in 10 plates). The plate contents are combined vertically, the reacted antibodies are separated and analyzed simultaneously, with de-convolution to determine the analyte concentration in the corresponding well of each plate.

### Examples 17. Detection of tagged proteins using 2D gel and mass spectrometry.

In this example, the ICP-DRC-MS technique is used in conjunction with the laser ablation of polyacrylamide gels containing proteins tagged by iron..It is well know that ArN⁺ and ArO⁺ interfere with ⁵⁴Fe⁺ and ⁵⁶Fe⁺, respectively. To facilitate the method described in Example 11, it is essential to remove isobaric poly-atomic interferences from the iron isotopes. For example, the ratio of the mass spectrometric signals at m/z=54:m/z=56 (where m/z indicates the mass-to-charge ratio of the ion) measured directly by ablation of the polyacrylamide gel containing a protein band tagged by iron was found to be 1.14 (whereas the expected value, based on the natural abundance of the iron isotopes, is 0.063). Utilizing ammonia as a reaction gas in the DRC environment, it is possible to eliminate ArN⁺ and ArO⁺ interferences by charge transfer reaction. This approach yielded the m/z=54:m/z=56 ratio that approximated the expected ⁵⁴Fe⁺/⁵⁶Fe⁺ isotope ratio, by which agreement the determination of the tag iron can be confirmed. In addition, the precision of this measurement is significantly improved due to partial temporal equilibration of ions in the gaseous media of the reaction cell (see Bandura, D.R., *et al.* 2000).

### REFERENCES

Baldwin, G.S., Hollande, F., Yang, Z., Karelina, Y., Peterson, A. Strang, R, Fourmy, D., Neumann, G., Shulkes, A. 2001 Biologically active recombinant human progastrin 6-80 contains a tightly bound calcium ion. J. Biol. Chem 276: 7791
Bandura D.R., Baranov V.I. and Tanner S.D. 2000. Effect of collisional damping and reactions in a dynamic reaction cell on the precision of the isotope ratio measurements. J.Anal. At. Spectrom., 15:921.
Baranov V.I., Tanner S.D. 1999 A dynamic reaction cell for inductively coupled plasma mass spectrometry (ICP-DRC-MS). Part I. The rf-field energy contribution in thermodynamics of ion-molecule reactions. J.Anal. At. Spectrom., 14:1133.
Barlett, P. A. et al, 1978 Synthesis of Water-Soluble Undecagold Cluster Compounds J. Am. Chem. Soc., 100: 5085
Binet M.R.B., Ma, R., McLeod, C. and Poole, R.K. 2001 Detection of zinc/cadmium binding proteins in E. coli by gel electrophoresis-laser ablation-inductively coupled plasma-mass spectrometry. Society for Experimental Biology Annual Meeting April2-6 C1.158 Canterbury, UK
Bio-Rad PLATELIA^{™} BSE test (DAS-ELISA) Blake, D.A., Blake 11, R.C., Khosraviani, M., Pavlov, A.R. 1998 Immunoassays for metal ions. Analytica Chimica Acta 376: 13
Bordes, A.L., Schollborn, B., Limoges, B., and Degrand, C. 1999. Simultaneous detection of three drugs labelled by cationic metal complexes at a nafion-loaded carbon paste electrode. Talanta 48: 201.
Bosslet, K., Hermentin, P., Seemann, G. 1999 Monoclonal antibody against complexed and non-complexed complexing agents for removing heavy metals from aqueous solutions and for analysis. US 5,907,034
Chen , X. Smith, L., Bradbury, E. 2000 Site-specific mass tagging with stable isotopes in proteins for accurate and efficient protein identification. Anal. Chem. 72: 1134-1143.
Cotton, F.A. and Wilkinson, G. 1972. Advanced Inorganic Chemistry, Interscience Publishers 528-530.
Darwish I.A. and Blake, D.A. 2001 One-step competitive immunoassay for cadmium ions: development and validation for environmental water samples. Analytical Chemistry 73: 1889
de Llano, J., Andreu, E., and Knecht, E. 1996. Use of Inductively Coupled Plasma-Mass Spectrometry for the Quantitation of the Binding and Uptake of Colloidal Gold-Low Density Lipoprotein Conjugates by Cultured Cells. Analytical Biochm. 243: 210-217.
de Llano, J., Andreu, E., Pastor, A., dela Guardia, M., Knecht, E. 2000. Electrothermal atomic absorption spectrometric diagnosis of familial hypercholesterolemia. Anal. Chem. 72: 2406-2413.
Frackelton, Jr., Raymond, A., Eisen, H., Ross, 1985 A. Production and use of monoclonal antibodies to phosphotyrosine-containing proteins. US 4,543,439.
Gillis, S 1983 Hybridoma antibody which inhibits interleukin 2 activity. US 4,411,993.
Hainfeld, J., Leone, R., Furuya, F., Powell, R. 1996 Small organometallic probes. US 5,521,289
Harlow and Lane (Eds) 1988 Antibodies: A Laboratroy Manual , Cold Spring Laboratroy Press
Hinshaw, J., Toner, J., Reynolds, G., 1987 Fluorescent labels fro immunoassay. US 4,637,988
Josel, H-P., Hoss, E., Ofenloch-Hahnle, B., Seidel, C., Upmeier, B., Wienhues, U-H. 1999 Metal complexes with a charged linker. US 5,958,783
Kennet, McKearn, Bechtold (eds) 1980 Monoclonal antibodies hybridomas: A new dimension in biological analyses. Plenum Press.
Kohler, G and Milstein, C. 1975. Continuous cultures of fused cells secreting antibody of predefined specificity. Nature 256: 495.
Korth, C., Stierli, B., Streit, P., Moser, M., Schaller, O., Fischer, R., Schulz-Schaeffer, W., Kretzschmar, H., Raeber, A., Braun, U., Ehrensperger, F., Hornemann, S., Glockshuber, R., Riek, R., Billerter, M., Wuthrich, K., and Oesch, B. 1997. Prion (PrPSc)-specific epitope defined by a monoclonal antibody. Nature, 390(6655), 74-7.
Laffling, A.J., Baird, A., Birkett, C.R., and John, H.A. 2001. A monoclonal antibody that enables specific immunohistological detection of prion protein in bovine spongiform encephalopathy cases. Neurosci. Lett. 300(2), 99-102.
Nagaoka, M. and Maitani, T. 2000 Binding patterns of co-existing aluminium and iron to human serum transferrin studied by HPLC-high resolution ICP-MS. Analyst 135: 1962-1965.
Qiu, J., and Song, J. F. 1996 A rapid polarographic immunoassay based on the anodic current of metal labelling. Anal. Biochem. 240: 13-16.
Segond von Banchet, G., and Heppelmann, B. 1995 Non-radioactive localization of substance P binding sites in rat brain and spinal cord using peptides labeled with 1.4 nm gold particles. J.Histochem.Cytochem. 43: 821-827.
Tanner S.D., Baranov V.I. 1999 A dynamic reaction cell for inductively coupled plasma mass spectrometry (ICP-DRC-MS). Part II. Reduction of interferences produced within the cell. JASMS, 101083.
Tanner S.D., Baranov V.I. and Vollkopf U. 2000a A dynamic reaction cell for inductively coupled plasma mass spectrometry (ICP-DRC-MS). Part III. Optimization and analytical performance; J.Anal. At. Spectrom., 15: 1261.
Tanner, S., Baranov, V. 2000b Bandpass reactive collision cell. US 6,140,638.
Wagenknecht, T., Berkowitz, J., Grassucci, R., Timerman. A.P., and Fleischer, S. 1994 Localization of calmodulin binding sites on the ryanodine receptor from skeletal muscle by electron microscopy. Biophys. J. 67: 2286-2295.
Wakabayashi, K., Sumi, Y., Ichikawa, Y., Sakato, Y., Mimuro, J., Aoki, N. 1990 Monoclonal antibody to human protein C. US 4,902,614
Wang, C. 1984 Method of Tagged Immunoassay. US 4,454,233.
Wenzel, T., and Baumeister, W. 1995 Conformational constraints in protein degradation by the 20S proteasome. Nature Structural Biol. 2: 199-204.
Wind, M., Edler, M., Jakubowski, N., Linscheid, M., Wesch, H., Lehmann, W. 2001 Analysis of protein phosphorylation by capillary liquid chromatography coupled to element mass spectrometry with P31 detection and to electrospray mass spectrometry. Anal. Chem. 73: 29-35.

## Claims

1. A method for the simultaneous detection and measurement of at least two isotopes of an element in a sample, where the measured isotopes are tags on two or more biologically active materials that bind with two or more analytes in the sample, comprising:
i) directly tagging the biologically active materials with isotopes of a transition element;
ii) combining the tagged biologically active materials with the analytes;
iii) separating bound tagged biologically active materials from unbound tagged materials;
iv) introducing two or more bound tagged biologically active materials having distinguishable isotopic tags into a sample of interest for simultaneous determination; and
v) simultaneously detecting and measuring the bound tagged isotopes by an inductively coupled plasma mass spectrometer.

2. The method of claim 1 wherein the analyte is a cell.

3. The method of one or more of the preceding claims wherein the tagged biologically active material or the tagged antibody is a commercially available product.

4. The method of one or more of the preceding claims, wherein the element is an isotope or ion.

5. The method of one or more of the preceding claims, wherein the element is selected from a group consisting of the noble metals, lanthanides, rare earth elements, gold, silver, platinum, rhodium, iridium and palladium.

6. The method of claim 5 wherein the element is one of gold and silver.

7. The method of one or more of the preceding claims wherein the step of tagging involves covalently coupling the element to one of the biologically active material and analyte.

8. The method of one or more of the preceding claims wherein the biologically active material is selected from a group consisting of an antibody, antigen, hormone, growth factor, receptor, protein and nucleic acid.

9. The method of one or more of the preceding claims wherein the tag includes more than one element.

10. The method of one or more of the preceding claims wherein the tag includes more than one isotope.

11. The method of one or more of the preceding claims wherein the tag includes more than one atom of an isotope.

12. The method of one or more of the preceding claims wherein the tag includes a different number of atoms of each isotope.

13. The method of one or more of the preceding claims comprising an additional step of sample introduction, wherein the sample introduction includes laser ablation.

14. The method of one or more of the preceding claims wherein the sample introduction includes laser ablation of polyacrylamide or agarose gels containing biologically active materials or analytes tagged by at least one element.

15. The method of one or more of the preceding claims wherein the sample introduction includes laser ablation of polyacrylamide or agarose gels containing biologically active materials or analytes tagged by atoms of at least one element having an un-natural isotopic composition.

16. The method of one or more of the preceding claims wherein the sample introduction includes laser ablation of animal tissue samples.

17. The method of one or more of the preceding claims wherein the sample introduction includes laser ablation of cell cultures.

18. The method of one or more of the preceding claims wherein the separating is selected from the group consisting of filtration, gel filtration, chromatography, electrophoreses, polyacrylamide gel electrophoreses, fractionation, absorption, agglutination and combinations thereof.

19. Use of an an inductively coupled plasma mass spectrometer in a method according to any one of claims 1 to 18.

## Patentansprüche

1. Verfahren zum simultanen Nachweis und zur Messung zumindest zweier Isotopen eines Elements in einer Probe, wobei die gemessenen Isotopen Marker an zwei oder mehr biologisch aktiven Materialien sind, die mit zwei oder mehreren Analyten in der Probe binden, umfassend:
i) direktes Markieren der biologisch aktiven Materialien mit Isotopen eines Übergangselementes;
ii) Kombinieren der markierten biologisch aktiven Materialien mit den Analyten;
iii) Trennen der gebundenen markierten biologisch aktiven Materialien von ungebundenen markierten Materialien;
iv) Einbringen von zwei oder mehr gebundenen biologisch aktiven Materialien, die unterscheidbare Isotopen-Markierungen aufweisen, in eine Probe von Interesse zur simultanen Bestimmung; und
v) simultanes Nachweisen und Messen der gebunden markierten Isotope durch ein induktiv gekoppeltes Plasma-Massenspektrometer.

2. Verfahren nach Anspruch 1, wobei der Analyt eine Zelle ist.

3. Verfahren nach einem oder mehreren der vorhergehenden Ansprüche, wobei das markierte biologisch aktive Material oder der markierte Antikörper ein kommerziell erhältliches Produkt ist.

4. Verfahren nach einem oder mehreren der vorhergehenden Ansprüche, wobei das Element ein Isotop oder Ion ist.

5. Verfahren nach einem oder mehreren der vorhergehenden Ansprüche, wobei das Element aus einer Gruppe ausgewählt ist, die aus Edelmetallen, Lanthaniden, Seltenerdelementen, Gold, Silber, Platin, Rhodium, Iridium und Palladium besteht.

6. Verfahren nach Anspruch 5, wobei das Element Gold oder Silber ist.

7. Verfahren nach einem oder mehreren der vorhergehenden Ansprüche, wobei der Schritt des Markierens eine kovalente Kopplung des Elementes an eines des biologisch aktiven Materials und des Analyten einschließt.

8. Verfahren nach einem oder mehreren der vorhergehenden Ansprüche, wobei das biologisch aktive Material aus einer Gruppe ausgewählt ist, die aus einem Antikörper, Antigen, Hormon, Wachstumsfaktor, Rezeptor, Protein und Nukleinsäure besteht.

9. Verfahren nach einem oder mehreren der vorhergehenden Ansprüche, wobei der Marker mehr als ein Element einschließt.

10. Verfahren nach einem oder mehreren der vorhergehenden Ansprüche, wobei der Marker mehr als ein Isotop einschließt.

11. Verfahren nach einem oder mehreren der vorhergehenden Ansprüche, wobei der Marker mehr als ein Atom eines Isotops einschließt.

12. Verfahren nach einem oder mehreren der vorhergehenden Ansprüche, wobei der Marker eine unterschiedliche Anzahl von Atomen jedes Isotops einschließt.

13. Verfahren nach einem oder mehreren der vorhergehenden Ansprüche, das einen zusätzlichen Schritt der Probeneinbringung umfasst, wobei die Probeneinbringung eine Laserablation einschließt.

14. Verfahren nach einem oder mehreren der vorhergehenden Ansprüche, wobei die Probeneinbringung eine Laserablation von Polyacrylamid oder Agarosegelen einschließt, die biologisch aktive Materialen oder Analyten enthalten, die durch zumindest ein Element markiert sind.

15. Verfahren nach einem oder mehreren der vorhergehenden Ansprüche, wobei die Probeneinbringung eine Laserablation von Polyacrylamid oder Agarosegelen einschließt, die biologisch aktive Materialen oder Analyten enthalten, die durch Atome zumindest eines Elementes mit einer unnatürlichen Isotopen-Zusammensetzung markiert sind.

16. Verfahren nach einem oder mehreren der vorhergehenden Ansprüche, wobei die Probeneinbringung eine Laserablation von Tiergewebsproben einschließt.

17. Verfahren nach einem oder mehreren der vorhergehenden Ansprüche, wobei die Probeneinbringung eine Laserablation von Zellkulturen einschließt.

18. Verfahren nach einem oder mehreren der vorhergehenden Ansprüche, wobei das Abtrennen aus der Gruppe ausgewählt ist, die aus Filtration, Gelfiltration, Chromatographie, Elektrophorese, Polyacrylamidgelelektrophorese, Fraktionierung, Absorption, Agglutination und Kombinationen hiervon besteht.

19. Verwendung eines induktiv gekoppelten Plasma-Massenspektrometers in einem Verfahren nach einem der Ansprüche 1 bis 18.

## Revendications

1. Procédé pour la détection et la mesure simultanées d'au moins deux isotopes d'un élément dans un échantillon, où les isotopes mesurés sont des marqueurs de deux ou plusieurs matières biologiquement actives qui se lient avec deux ou plusieurs analytes dans l'échantillon, comprenant :
i) le marquage direct des matières biologiquement actives avec les isotopes d'un élément de transition ;
ii) la combinaison avec les analytes des matières biologiquement actives marquées ;
iii) la séparation des matières biologiquement actives marquées liées des matières marquées non liées ;
iv) l'introduction de deux ou plusieurs matières biologiquement actives marquées liées ayant des marqueurs isotopiques pouvant être distingués dans un échantillon d'intérêt pour la détermination simultanée ; et
v) la détection et la mesure simultanées des isotopes marqués liés par un spectromètre de masse à plasma couplé par induction.

2. Procédé selon la revendication 1, dans lequel l'analyte est une cellule.

3. Procédé selon une ou plusieurs des revendications précédentes, dans lequel la matière biologiquement active marquée ou l'anticorps marqué est un produit disponible dans le commerce.

4. Procédé selon une ou plusieurs des revendications précédentes, dans lequel l'élément est un isotope ou un ion.

5. Procédé selon une ou plusieurs des revendications précédentes, dans lequel l'élément est sélectionné dans un groupe constitué par les métaux nobles, les lanthanides, les éléments des terres rares, l'or, l'argent, le platine, le rhodium, l'iridium et le palladium.

6. Procédé selon la revendication 5, dans lequel l'élément est l'un parmi l'or et l'argent.

7. Procédé selon une ou plusieurs des revendications précédentes, dans lequel l'étape de marquage implique le couplage par covalence de l'élément à l'une de la matière biologiquement active et de l'analyte.

8. Procédé selon une ou plusieurs des revendications précédentes, dans lequel la matière biologiquement active est sélectionnée dans un groupe constitué par un anticorps, un antigène, une hormone, un facteur de croissance, un récepteur, une protéine et un acide nucléique.

9. Procédé selon une ou plusieurs des revendications précédentes dans lequel le marqueur inclut plus d'un élément.

10. Procédé selon une ou plusieurs des revendications précédentes, dans lequel le marqueur inclut plus d'un isotope.

11. Procédé selon une ou plusieurs des revendications précédentes, dans lequel le marqueur inclut plus d'un atome d'un isotope.

12. Procédé selon une ou plusieurs des revendications précédentes dans lequel le marqueur inclut un nombre différent d'atomes de chaque isotope.

13. Procédé selon une ou plusieurs des revendications précédentes comprenant une étape supplémentaire d'introduction d'un échantillon, dans lequel l'introduction d'échantillon inclut une ablation au laser.

14. Procédé selon une ou plusieurs des revendications précédentes dans lequel l'introduction d'échantillon inclut l'ablation au laser de gels de polyacrylamide ou d'agarose contenant des matières biologiquement actives ou des analytes marquées par au moins un élément.

15. Procédé selon une ou plusieurs des revendications précédentes dans lequel l'introduction d'échantillon inclut l'ablation au laser de gels de polyacrylamide ou d'agarose contenant des matières biologiquement actives ou des analytes marquées par des atomes d'au moins un élément ayant une composition isotopique non naturelle.

16. Procédé selon une ou plusieurs des revendications précédentes dans lequel l'introduction d'échantillon inclut l'ablation au laser d'échantillons de tissus animaux.

17. Procédé selon une ou plusieurs des revendications précédentes, dans lequel l'introduction d'échantillon inclut l'ablation au laser de cultures de cellules.

18. Procédé selon une ou plusieurs des revendications précédentes, dans lequel la séparation est sélectionnée dans le groupe constitué par la filtration, la filtration sur gel, la chromatographie, les électrophorèses, les électrophorèses en gel de polyacrylamide, le fractionnement, l'absorption, l'agglutination et des combinaisons de ces derniers.

19. Utilisation d'un spectromètre de masse à plasma couplé par induction dans un procédé selon l'une quelconque des revendications 1 à 18.
